# FASCICULE DE BREVET EUROPEEN

(11) **EP 3 171 956 B1**
(45) Date de publication et mention de la délivrance du brevet: **29.04.2020**
(21) Numéro de dépôt: 15753115.3
(22) Date de dépôt: 17.07.2015
(51) Int. Cl.: B01D 11/04, B01D 15/00, C12P 7/40, C12P 7/52, C12P 7/54

(54) **PROCEDE D'EXTRACTION DE MOLECULES PRODUITES PAR FERMENTATION ANAEROBIE A PARTIR DE BIOMASSE FERMENTESCIBLE**
VERFAHREN ZUR EXTRAKTION VON MOLEKÜLEN DURCH ANAEROBE GÄRUNG AUS VERGÄRBARER BIOMASSE
METHOD FOR EXTRACTING MOLECULES PRODUCED BY ANAEROBIC FERMENTATION FROM FERMENTABLE BIOMASS

(30) Priorité: 25.07.2014 FR 1457201
(43) Date de publication de la demande: 31.05.2017
(73) Titulaire: Afyren, 63360 Saint-Beauzire (FR)
(72) Inventeur: NOUAILLE, Régis, F-63800 Cournon d'Auvergne (FR); PESSIOT, Jérémy, F-58400 La Charite sur Loire (FR)
(74) Mandataire: Dennemeyer & Associates S.A.
(86) Numéro de dépôt international: PCT/FR2015/051964
(87) Numéro de publication internationale: WO 2016/012698

(56) Documents cités:
- EP-A2- 0 216 221
- WO-A2-2013/022998
- FR-A1- 2 588 271
- US-A- 4 424 275
- HUANG H J ET AL: "A review of separation technologies in current and future biorefineries", SEPARATION AND PURIFICATION TECHNOLOGY, ELSEVIER SCIENCE, AMSTERDAM, NL, vol. 62, no. 1, 1 août 2008 (2008-08-01), pages 1-21, XP022710820, ISSN: 1383-5866, DOI: 10.1016/J.SEPPUR.2007.12.011 [extrait le 2007-12-27]
- Shang-Tian Yang ET AL: "Extraction-Fermentation Hybrid (Extractive Fermentation)" In: "Separation and Purification Technologies in Biorefineries", 11 février 2013 (2013-02-11), John Wiley & Sons, Ltd, Chichester, UK, XP055177441, ISBN: 978-0-47-097796-5 pages 409-437, DOI: 10.1002/9781118493441.ch15, pages 419-421; tableau 15.5

## Description

La présente invention concerne un procédé d'extraction de molécules produites par fermentation anaérobie à partir de biomasse fermentescible.

Par biomasse fermentescible, on désigne ici un substrat organique, avantageusement non alimentaire, obtenu à partir de déchets, sous-produits et coproduits formés de matières organiques, c'est-à-dire de la biomasse, issue des activités humaines, qu'elles soient domestiques, industrielles, agricoles, forestières, aquacoles, agro-industrielles, ou issue de l'élevage. A titre d'exemple non limitatif, on peut citer comme substrat organique les fumiers, la fraction organique des ordures ménagères, les coproduits d'abattoir, des résidus cellulosiques ou ligno-cellulosiques provenant de l'agro-industrie tels ceux issus de la transformation de la canne à sucre (bagasse), du tournesol ou du soja.

Par fermentation anaérobie on entend une fermentation réalisée dans des conditions anaérobies par des microorganismes, eucaryotes ou procaryotes, tels que des bactéries, des champignons, des algues ou des levures.

Le terme molécule désigne ici, préférentiellement mais non exclusivement, des métabolites fermentaires dits précurseurs. Ces précurseurs permettent par la suite la production de molécules qui présentent un plus grand intérêt énergétique et/ou chimique, étant entendu qu'il s'agit de molécules organiques. On peut citer comme molécules ayant un intérêt énergétique et/ou chimique, par exemple, des molécules ayant une chaine carbonée telles que des acides, des hydrocarbures, du méthane, des esters, des alcools, des amides ou des polymères.

Parmi les métabolites fermentaires dits précurseurs, produits lors de la fermentation, on peut citer les acides gras volatils ou AGV qui peuvent être convertis, par exemple, en cétones, en alcanes, alcools, alcènes, étant entendu qu'une telle fermentation produit également, entre autres, des esters, des gaz, de l'acide lactique, des alcools, de l'hydrogène et du dioxyde de carbone.

US-A-6 043 392 décrit un tel procédé permettant de produire des cétones par un traitement thermique des sels d'acides gras volatils obtenus par fermentation anaérobie. Une partie des acides gras volatils est également convertie en hydrocarbures liquides, en aldéhydes et en alcools. Il s'avère que ce procédé s'effectue en deux étapes distinctes, à savoir la fermentation puis le traitement des AGV par extraction sous forme de sels précipités avec un extradant de type amine tertiaire. Il est par ailleurs connu que la production d'acides gras volatils effectuée par une fermentation anaérobie induit une acidification du milieu préjudiciable aux microorganismes. L'acidification du milieu induisant une inhibition des microorganismes, donc un ralentissement voir un arrêt de la fermentation, il est nécessaire de travailler en discontinu. Pour cela, les AGV sont extraits après un temps de fermentation donné, par des techniques connues en soi. Le procédé ne permet donc pas une production rapide et en continue de molécules dites précurseurs, le rendement n'étant pas optimal. On connait par US-A-4 424 275 une fermentation anaérobie destinée à produire du butanol. L'extraction est réalisée en continue avec un solvant contenant du chlore et du fluor. EP-A-216 221 divulgue une extraction liquide/liquide associée à une fermentation avec un solvant non toxique pour les microorganismes. De tels procédés sont consommateurs de souches de microorganismes et génèrent des déchets peu ou pas valorisables.

L'invention vise à proposer un autre procédé d'extraction permettant de produire de manière continue, biocompatible, régulière, maitrisée et avec un minimum de déchets peu valorisables, diverses molécules, dites précurseurs, obtenues par une fermentation anaérobie.

A cet effet, l'invention a pour objet un procédé d'extraction d'acides gras volatils (AGV), molécules organiques dites précurseurs produites par des microorganismes dans un réacteur de fermentation par fermentation anaérobie à partir de biomasse fermentescible, lesdites molécules étant des métabolites fermentaires, caractérisé en ce qu'il comprend au moins les étapes suivantes :
- a) choisir un moyen d'extraction parmi des moyens d'extraction qui sont, au moins, insolubles dans le milieu de fermentation et dont les conditions de mise en œuvre préservent la capacité des microorganismes présents dans le milieu de fermentation à produire les molécules, le moyen d'extraction est un solvant dont le point d'ébullition est inférieur à 70°C, l'extraction étant de type liquide-liquide et le moyen d'extraction est un solvant ayant un point d'ébullition inférieur à la température de fermentation,
- b) mettre en contact le moyen d'extraction choisi avec le milieu de fermentation, sans interruption de la fermentation,
- c) récupérer les molécules extraites, à un pH inférieur à 4,5, par le moyen d'extraction en dehors du réacteur de fermentation
- d) après l'étape c) au moins une partie de la phase liquide issue de l'extraction est réintroduite dans le réacteur de fermentation et incorporée au milieu de fermentation.

Un tel procédé permet d'extraire, en continu, des molécules en particulier des métabolites fermentaires dits précurseurs tels que des acides gras volatils, tout en préservant la capacité de production des microorganismes présents dans le bioréacteur. En effet, l'étape d'extraction permet non seulement de collecter en continu les molécules produites dans le réacteur de fermentation mais également de préserver les microorganismes responsables de cette production, l'extraction étant effectuée dans des conditions non létales pour la totalité des microorganismes, c'est-à-dire dans des conditions d'extraction biocompatibles. De cette manière, on s'affranchit des problèmes liés à l'accumulation des précurseurs dans le réacteur de fermentation, par exemple de l'acidification du milieu de fermentation par accumulation des acides gras volatils produits qui sont nocifs pour les microorganismes. On maintient à un niveau élevé, proche du niveau initial, l'activité des microorganismes tout au long du cycle de fermentation.

Selon des aspects avantageux mais non obligatoires de l'invention, un tel procédé peut comprendre une ou plusieurs des caractéristiques suivantes:
- Le moyen d'extraction est un solvant dont la densité est inférieure à la densité du milieu de fermentation.
- La mise en contact entre le milieu de fermentation et le moyen d'extraction a lieu dans le réacteur, le moyen d'extraction étant isolé ou non du milieu de fermentation.
- La mise en contact entre le milieu de fermentation et le moyen d'extraction a lieu hors du réacteur, le milieu de fermentation étant prélevé en continu.
- La mise en contact entre le milieu de fermentation et le moyen d'extraction a lieu hors du réacteur, le milieu de fermentation étant prélevé séquentiellement.
- Après l'étape c) au moins une partie de la phase liquide issue de l'extraction et réintroduite dans le réacteur de fermentation et incorporée au milieu de fermentation.
L'invention concerne également une Installation de de mise en œuvre d'un procédé conforme à l'une des caractéristiques précédentes, caractérisée en ce qu'elle comprend au moins :
- un réacteur de fermentation,
- un organe d'extraction propre à assurer la mise en contact entre le milieu de fermentation et le moyen d'extraction.

L'invention sera mieux comprise et d'autres avantages de celle-ci apparaitront plus clairement à la lecture de la description de plusieurs modes de réalisation de l'invention, donnée à titre d'exemple non limitatif et faite en référence aux dessins suivants dans lesquels:

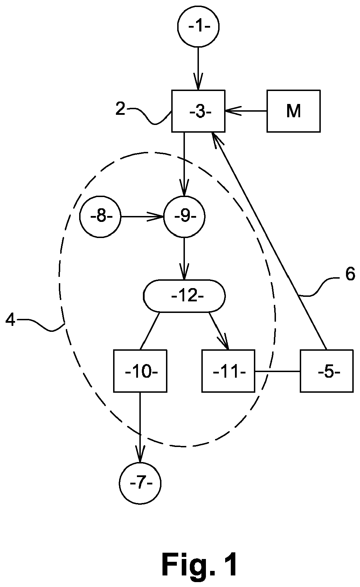

- La figure 1 est un schéma simplifié représentatif du procédé objet de l'invention.

Les différentes étapes du procédé sont maintenant décrites en référence à plusieurs modes de réalisation, étant entendu que les étapes connues en soi ne sont pas détaillées. En particulier, il sera fait référence par la suite au diagramme de la figure 1 comme illustrant un mode avantageux de réalisation de l'invention.

Tout d'abord le substrat 1 utilisé est, avantageusement, non traité, à savoir qu'il n'a subi aucun prétraitement physico-chimique ou enzymatique. Ce substrat 1 est majoritairement constitué par de la biomasse fermentescible. A titre d'exemple non limitatif, on peut citer les déchets agricoles ou végétaux (paille, bagasse, drèche de maïs, herbes, bois, tontes) les déchets papetiers (carton, papier), les déchets agroalimentaires, les déchets d'abattoirs, la fraction organique des ordures ménagères, les effluents d'élevage (fumiers, lisiers, fientes), les algues, les déchets d'aquaculture, les déchets d'activité forestière ou encore les coproduits fermentescibles de l'industrie cosmétique. Certains substrats contiennent des molécules organiques, telles des acides organiques, qui n'influeront pas, ou de façon marginale, sur le procédé de fermentation. En revanche, ces molécules peuvent se retrouver dans le milieu de fermentation et participer, par exemple, à la production des molécules organiques finales définies.

Le substrat 1 est introduit dans un réacteur de fermentation 2, connu en soi et dimensionné pour la production souhaitée, que cette dernière soit à l'échelle du laboratoire pour effectuer des essais ou à l'échelle industrielle dans le cas d'une production. En d'autres termes, le réacteur de fermentation 2 ou bioréacteur a un volume variant de quelques litres à plusieurs centaines de mètres cubes, selon les besoins.

Des microorganismes sont, avantageusement, introduits initialement dans le réacteur de fermentation, en quantité suffisante pour débuter la fermentation. Les microorganismes sont, avantageusement, inoculés sous forme d'un consortium, illustré par la flèche M. Par le terme consortium, on désigne un mélange ou mix de microorganismes, eucaryotes et procaryotes, qu'il s'agisse de bactéries, levures, champignons ou algues. Ces microorganismes proviennent essentiellement d'écosystèmes naturels, avantageusement mais non exclusivement d'écosystèmes anaérobies tels que, à titre d'exemple non limitatif, la zone anaérobie des milieux aquatiques telle la zone anoxique de certains lacs, les sols, les marais, les boues d'épuration, le rumen des ruminants ou l'intestin des termites. Il convient de garder à l'esprit que la distribution qualitative et quantitative des différents types et espèces de microorganismes dans le consortium M n'est pas connue précisément et surtout peut varier dans des proportions importantes. Il s'avère que cette diversité qualitative et quantitative apporte de façon surprenante une robustesse et une adaptabilité des microorganismes qui permettent d'assurer une utilisation optimale des substrats, quel que soit la composition de ces derniers et cela dans des conditions de fermentation variables.

Par ailleurs, du fait que le substrat 1 est utilisé tel quel, c'est-à-dire qu'il n'est pas stérilisé ou, plus généralement, qu'il n'est pas débarrassé des microorganismes qu'il contient préalablement à son introduction dans le bioréacteur 2, il s'avère que les microorganismes endémiques au substrat 1 sont, de facto, incorporés dans le consortium M ou du moins associés à ce dernier dans le bioréacteur 2.

Le consortium M de microorganismes, associé aux microorganismes éventuellement présents dans le substrat 1, permet la fermentation 3 du substrat 1, cela sans ajout de produits tels que des enzymes. Par ailleurs, la fermentation 3 a lieu en conditions anaérobies, plus précisément lorsque le potentiel redox est inférieur à -300mV, avantageusement compris entre -550mV et -400mV et lorsque le pH est inférieur à 8, de préférence compris entre 4 et 7. La fermentation 3 est, avantageusement, limitée à la production de métabolites fermentaires dits précurseurs, à savoir des acides gras volatils ou AGV. On induit ainsi une réaction similaire au phénomène d'acidose rencontré chez les ruminants tout en ayant une production de méthane proche de zéro. Le méthane est, généralement, un des métabolites fermentaires finaux obtenus lors d'une fermentation anaérobie par des microorganismes issus d'écosystèmes naturels.

La fermentation 3 conduit, dans un premier temps, à la formation d'acides gras volatils ayant de un à huit carbones, principalement de deux à quatre carbones tels que l'acide acétique, l'acide propionique et l'acide butyrique. On obtient aussi des acides gras volatils à longue chaine, donc supérieure à quatre carbones, tels que les acides valérique et caproïque, heptanoïque ou octanoïque. En poursuivant la fermentation et/ou en augmentant la quantité de microorganismes dans le bioréacteur 2, si besoin avec des microorganismes sélectionnés, il est possible de favoriser la production d'AGV à longue chaine carbonée, donc supérieure à quatre carbones.

En d'autres termes, les métabolites produits en quantité lors de la fermentation 3 sont essentiellement des acides gras volatils de deux à six carbones. Par la suite, l'extraction concernera essentiellement l'extraction de ces métabolites, étant entendu que le procédé peut être mis en oeuvre pour d'autres molécules produites lors d'autres types de fermentation. La fermentation 3 peut être conduite en mode discontinu ou batch, en continu-discontinu ou fed-batch ou, de façon préférée, encore en continu dans un seul ou dans plusieurs réacteurs de fermentation disposés en série.

La fermentation 3 est, dans tous les cas, menée pour assurer la production de molécules données, donc d'AGV, en phase liquide, étant entendu que l'invention concerne tout type de molécules organiques produites par fermentation ou métabolites, pour autant qu'elles soient produites en phase liquide. Ainsi, on conçoit aisément que le milieu de fermentation comprend une phase solide contenant, au moins initialement, la fraction solide du substrat 1 ainsi que la fraction solide du consortium M de microorganismes. La phase liquide du milieu de fermentation contient les molécules produites lors de la fermentation 3 ainsi que la fraction liquide du substrat 1, au moins lors du début de fermentation.

Le temps de fermentation varie, entre autres, en fonction du substrat 1, des microorganismes M présents et des conditions de fermentation. Typiquement, la période de fermentation est comprise entre 1 et 7 jours, préférentiellement entre 2 et 4 jours. La concentration en métabolites obtenue dans le milieu de fermentation à l'issue de cette période est variable, mais, par exemple pour des acides gras volatils, est généralement de l'ordre de 10 à 20 g/L, selon les acides gras volatils, étant entendu que dans certaines conditions elle peut être supérieure à 35 g/L, par exemple voisine de 50 g/L. A la fin de l'étape de fermentation 3, le milieu de fermentation est à un pH acide, qui est généralement compris entre 4 et 6, du fait de la présence des acides gras volatils dans le milieu de fermentation.

Lorsque la production en métabolites, ou molécules, prédéfinis, ici en AGV, par fermentation 3 du substrat 1 atteint une quantité définie, généralement en phase de régime permanent de la fermentation, l'étape d'extraction 4 des molécules est initiée. De manière préférée mais non obligatoire, cette quantité correspond à un ralentissement de la croissance des microorganismes, donc au voisinage d'un seuil d'inhibition des microorganismes.

Le moyen d'extraction est choisi parmi des moyens d'extraction, liquide ou solide, qui sont, au moins, insolubles dans le milieu de fermentation. Lorsque le moyen d'extraction est liquide, donc lorsqu'il s'agit d'un solvant, le point d'ébullition de ce dernier est, avantageusement, inférieur à 70°C. Préférentidlement, la densité du solvant est inférieure à celle du milieu de fermentation.

De manière plus précise, l'extraction 4 est conduite avec un moyen d'extraction 8, solide ou liquide, dont les conditions de mises en œuvre permettent de préserver l'activité et/ou la croissance des microorganismes M dans les conditions de fermentation régnant dans le bioréacteur 2 et définies pour réaliser la fermentation 3. Les molécules, donc ici les métabolites fermentaires, sont préférentiellement extraites individuellement, ou du moins extraites par familles moléculaires, à partir de la phase liquide du milieu de fermentation, ce qui permet entre autres des meilleurs rendements et facilite la production de composés spécifiques à partir de ces molécules extraites.

Dans tous les cas, les métabolites produits par la fermentation 3, ici anaérobie, et qui sont, au moins en partie, extraits le sont dans des conditions telles que l'extraction 4 ne détruit pas les microorganismes M, ou du moins dans des proportions telles que cela ne modifie pas de manière sensible la poursuite de la fermentation 3 par les microorganismes M présents dans le milieu de fermentation. En d'autres termes le moyen d'extraction 8 n'est pas létal pour la totalité des microorganismes. L'extraction 4 n'interfère donc pas, ni ne dégrade, le milieu de fermentation ni les capacités fermentaires des microorganismes M qu'il contient. L'extraction 4 est donc réalisée dans des conditions telles qu'elle est biocompatible.

Lorsque l'on extrait du milieu de fermentation des molécules telles que des acides gras volatils, de facto on réduit l'acidification du milieu de fermentation par ces acides. Ainsi, la fermentation, et donc la production de métabolites, se poursuit dans des conditions similaires aux conditions initiales, le milieu de fermentation restant peu acide.

De manière avantageuse, dans la mesure où la méthode d'extraction retenue n'est pas létale pour la totalité des microorganismes, il s'avère que la phase liquide résiduelle 5, après l'extraction 4, peut contenir des microorganismes M vivants, donc potentiellement actifs. Comme dans cette phase liquide 5 il y a moins d'acides gras volatils qu'initialement, le pH de la phase liquide 5 est moins acide. Il est donc possible de la réinjecter dans le réacteur de fermentation 2, comme illustré par la flèche 6. Ainsi, non seulement on diminue le phénomène d'acidose et/ ou on abaisse le pH du milieu de fermentation en cours de fermentation 3, par extraction 4 des composés acides, mais, dans une certaine mesure, on réensemence également le milieu avec des microorganismes assurant la fermentation 3, cela sans abaisser le pH du milieu de fermentation.

Une telle solution permet d'optimiser le rendement de la fermentation 3 et de réaliser une fermentation en continue, cela en abaissant les temps de fermentation, tout en tendant vers le zéro déchet.

L'extraction 4 est conduite en continue ou de manière séquentielle, par exemple avec une extraction toutes les 12 heures. En d'autres termes, il est possible de poursuivre la fermentation 3 tout en extrayant les métabolites produits, soit au fur et à mesure de leur production soit de manière régulière. Une fois extraits, les métabolites sont purifiés et/ou transformés en d'autres produits 7, tels que des alcanes, des alcènes, des amides, des amines, des esters, des polymères par des techniques chimiques connues en soi comme, par exemple, la distillation, la synthèse, l'électrosynthèse, l'amidation ou la polymérisation.

L'extraction liquide-liquide avec des solvants organiques polaires ou non polaires comme moyen d'extraction 8 est le mode d'extraction, préférentiellement mais non exclusivement, retenu.

De manière plus précise, lorsque l'extraction est de type liquide-liquide, on met en contact 9, préférentiellement sous agitation, un mélange formé du solvant organique 8 et de la phase liquide provenant du milieu de fermentation afin de faciliter le transfert de la phase aqueuse vers la phase organique.

Après la mise en contact 9, les phases organique 10 et aqueuse 11 sont, avantageusement, séparées par décantation 12. A l'issue de cette dernière, la phase aqueuse 11 est appauvrie en métabolites, ici en acides gras volatils, la phase organique 10 s'étant elle enrichie en métabolites, donc en acides gras volatils.

Dans un mode de réalisation, l'extraction n'est pas réalisée dans un organe distinct du réacteur de fermentation mais directement dans ce dernier. Le solvant étant, par exemple, introduit par un dispositif de type bulleur situé en partie basse du réacteur. En variante, l'organe d'extraction est inséré dans le volume du réacteur, une communication avec le milieu de fermentation étant ménagée.

Il est alors possible de réintroduire la phase aqueuse 11, donc éventuellement des microorganismes M, dans le bioréacteur 2.

Les métabolites dits précurseurs sont collectés à partir de la phase organique 10 par des techniques connues en soi, telles que la distillation ou l'évaporation. Avantageusement, le solvant organique 8 est choisi pour avoir un point d'ébullition bas et, dans tous les cas, dans une plage de températures non létales pour les microorganismes, à savoir inférieur à 70°C. En d'autres termes, le point d'ébullition du solvant est de préférence inférieur à la température de fermentation afin qu'il n'y ait pas de traces du solvant dans le milieu de fermentation.

De plus, l'évaporation d'un solvant à bas point d'ébullition est peu couteuse en énergie et préserve au mieux les molécules extraites, les températures atteintes pour évaporer le solvant ne générant aucune dégradation thermique des molécules à extraire.

En d'autres termes, le point d'ébullition du solvant organique est, avantageusement, également inférieur au point d'ébullition des molécules extraites.

Par ailleurs, une densité du solvant inférieure à la densité du milieu de fermentation permet d'obtenir, préférentiellement, un surnageant dans lequel les molécules sont présentes. Dans le cas où l'extraction est réalisée en dehors du bioréacteur 2, les éventuels microorganismes présents sont alors cantonnés dans la phase aqueuse et, par gravité, sont ainsi isolés du surnageant et donc du solvant 8. On limite ainsi au moins l'inhibition si ce n'est la destruction des microorganismes par le solvant.

Des essais ont été menés par la demanderesse selon différents modes de réalisation, dans le cas de l'extraction d'acides gras volatils.

### Essai 1 :

Les acides gras volatils produits au cours d'une fermentation qui est réalisée sur un substrat comprenant la fraction fermentescible des ordures ménagères à une concentration de 50 g/L de matière sèche (MS). 50 ml du milieu de fermentation, donc la phase liquide, sont récupérés. Le pH de ce prélèvement est de 4,3. Ces 50 ml sont soumis ensuite, sous agitation 9 modérée, à une extraction au pentane en tant que moyen d'extraction 8, mettant en oeuvre un volume égal de phase organique (soit 50 ml) et de phase aqueuse.

Après séparation des phases organique 10 et aqueuse 11 par décantation 12, on récupère, par évaporation du solvant, 14,3 g/L d'acides gras volatils dans la phase organique et 41,1 g/L d'acides gras volatils dans la phase aqueuse, soit un rendement d'extraction de 26%. 0,7 g d'acides gras volatils biosourcés, c'est-à-dire provenant de la fermentation 3 du substrat 1 sont récupérés.

### Essai 2 :

On répète l'essai 1 mais en acidifiant le prélèvement effectué dans le milieu de fermentation avec de l'acide chlorhydrique (HCl) à 10 M jusqu'à pH 2,25 au lieu de 4,3. On récupère de cette façon, après agitation 9, décantation 12 et évaporation du solvant, dans la phase organique 18 g/L d'AGV et 32,5 g/L d'AGV dans la phase aqueuse. Le rendement d'extraction obtenu dans ce cas est de 36 %.

### Essai 3 :

On répète l'essai 2 en utilisant le même milieu de culture mais en amenant le pH à 5,35 au lieu de 4,3 à l'aide d'une solution de NaOH à 10 M. On récupère de cette façon dans la phase organique 6 g/L d'AGV et 44,5 g/L d'AGV dans la phase aqueuse. Le rendement d'extraction obtenu dans ce cas est de 12 %.

### Essai 4 :

On répète l'essai 1 avec de l'éther diéthylique au lieu de pentane comme moyen d'extraction 8, et on étudie l'influence du pH sur le rendement d'extraction. Les rendements d'extractions obtenus pour une extraction à l'éther di-éthylique en ratio 1/1 à différents pH sont les suivants :

| pH | Rendement d'extraction |
|---|---|
| 1,5 | 55% |
| 4,3 | 43% |
| 6 | 8% |

Il apparait donc que les rendements obtenus sont différents suivant les conditions de pH de l'extraction, cela quel que soit le moyen d'extraction liquide 8 retenu, ici un solvant organique. En particulier, la demanderesse a constaté de manière surprenante qu'avec un pH supérieur à 4,5, les rendements chutent. Un pH inférieur au pH initial du milieu de fermentation, avantageusement un pH inférieur à 3 induira un meilleur rendement d'extraction comparé à un pH intermédiaire, étant entendu qu'un pH intermédiaire permettra un meilleur rendement d'extraction qu'avec un pH supérieur à 6. Ces résultats sont, de façon inattendue, également observés pour d'autres types de solvants comme illustré par les résultats de l'essai numéro 5.

### Essai 5 :

On étudie l'influence de la nature du solvant sur le rendement d'extraction avec un milieu acidifié à un pH inférieur à 3. Les résultats obtenus sont :

| Solvants | Rendement d'extraction |
|---|---|
| Pentane | 36% |
| Cyclopentane | 35% |
| Hexane | 33% |
| Cyclohexane | 24% |
| Heptane | 30% |

Il apparait que les rendements obtenus sont les plus élevés en utilisant des solvants comme le pentane, le cyclopentane ou l'éther diéthylique comme vu dans l'essai 4, étant entendu que les rendements sont dans tous les cas supérieurs à 20%. On conçoit que le moyen d'extraction peut être un mélange d'au moins deux solvants, pour autant que le mélange soit, au moins, insoluble dans le milieu de fermentation. Ces solvants sont de plus intéressants dans la mesure où leur point d'ébullition est bas, inférieur à 100°C et, plus spécifiquement, compris entre 30°C et 70°C. Cei permet une extraction des molécules directement dans le réacteur 2 sans modifier le mode mésophile ou thermophile. De plus, bien que ce type de solvant soit quasi insoluble dans l'eau, leur faible point d'ébullition permet une bonne séparation entre la phase organique et la phase aqueuse à la température rencontrée dans les réacteurs lors des fermentations.

La phase aqueuse qui contient alors une quantité moindre qu'initialement d'acides gars volatils et qui n'a pas été altérée par l'extraction 4 peut être recyclée, à savoir réintroduite dans le réacteur de fermentation, pour participer à la continuité de la fermentation 3.

Une série d'essais a été menée en modifiant le ratio solvant/soluté pour l'extraction liquide-liquide. On effectue l'extraction avec un ratio solvant/soluté en volume de 2/1, au lieu de 1/1, sur un milieu de fermentation similaire à celui du premier exemple. Un rendement d'extraction de 43 % contre 36 % dans l'exemple 1 est constaté.
On répète l'essai avec un ratio solvant/soluté en volume de 1/2. Le rendement d'extraction est de 27 % contre 36 % dans l'exemple 1.

On constate donc que le rendement d'extraction est amélioré en augmentant significativement la proportion de solvant par rapport au soluté, cela sans que les microorganismes soient affectés.

Avantageusement, la demanderesse a constaté qu'il est possible d'augmenter les rendements d'extraction de type liquide-liquide en utilisant, en combinaison avec le solvant ou le mélange de solvants, un ou des composés fréquemment désignés par le terme anglais extractant, c'est-à-dire un composé apte à réagir avec un soluté dans une solution comme entre autre le TBP (TriButylPhosphate). Avec une concentration de 10% de cet extractant sur un milieu de fermentation similaire à l'exemple 1, le rendement d'extraction est de 53% avec du pentane et de 50% avec de l'hexane contre 36% dans l'essai 1 sans extradant, les conditions de pH et de ratio étant similaires à celles de l'essai 1.

Des essais ont également été menés avec d'autres moyens d'extraction, en particulier des éléments d'extraction solides, dans le cadre d'une extraction de type solide-liquide. On peut citer des résines, du charbon actif ou des zéolithes comme tels moyens d'extraction.

Dans tous les cas, le moyen d'extraction solide est, avantageusement, le plus hydrophobe possible.

Un essai avec l'utilisation d'une résine anionique a permis un rendement d'extraction des acides gras volatils à partir d'un milieu de fermentation similaire à celui du premier essai de 22% en 15 minutes. Une fois l'étape d'extraction réalisée, ces moyens solides peuvent être régénérés et servir lors d'autres étapes d'extraction.

L'utilisation de charbon actif ayant déjà servi et qui a été régénéré afin d'extraire les acides gras volatils a été testée, sur un milieu de fermentation similaire à celui du premier essai. Une fois le milieu de fermentation diminué, par extraction sur le charbon actif, d'une partie des acides gras volatils totaux, celui-ci a été réutilisé pour de nouvelles fermentations anaérobies, avec différents consortia de microorganismes et sur différents substrats.

Les résultats obtenus, suite à une extraction des acides gras volatils, quel que soit le type d'extraction, montrent que les concentrations en acides gras volatils, pour toutes les fermentations, donc à partir de différents substrats et/ou consortia de microorganismes sont toutes supérieures à la concentration initiale en acides gras volatils dans le milieu avant fermentation et ce sur plusieurs générations de cultures. L'activité fermentaire a été maintenue en utilisant des milieux ayant subi des étapes d'extraction. Ceci montre que ce procédé d'extraction, liquide-liquide ou solide-liquide, n'altère pas les caractéristiques du milieu de fermentation et permet une production continue avec un recyclage, au moins partiel, de ce dernier.

Des essais d'extraction in situ ont permis de montrer la biocompatibilité du moyen d'extraction, autrement dit la récupération séquentielle ou en continu de molécules telles que des acides gras volatils produites par des microorganismes lors d'une fermentation d'un substrat sur plus de 2000 heures. Cette biocompatibilité est caractérisée par le nombre de microorganismes par ml présents dans le bioréacteur déterminée par la technique d'analyse de cytométrie en flux. Ces résultats sont, par exemple, entre des échantillons prélevés avant et après extraction in situ, de 2,3.10⁸ à 8,0.10⁷ microorganismes/ml, dans une série de mesures et de 2,9 à 2,3.10⁸ microorganismes/ml pour une autre série de mesures. Ceci montre qu'il y a une diminution de la population de microorganismes présents dans le bioréacteur, suite à l'extraction des molécules produites, donc en l'espèce des acides gras volatils, donc de facto suite à un prélèvement du milieu de fermentation, mais que cette diminution n'entraine pas de destruction massive et totale des microorganismes. La population en microorganismes est suffisante, quantitativement et qualitativement, pour que les microorganismes soient actifs et qu'il n'y ait pas, ou très peu, de perte de l'activité fermentaire du consortium de microorganismes. En d'autres termes, les fluctuations de la population de microorganismes, du fait de l'extraction, n'affectent pas, à l'échelle macroscopique, l'activité globale des microorganismes, ce qui permet de maintenir une production optimale de métabolites fermentaires dits précurseurs.

L'extraction peut donc être réalisée, sans contraintes irréversibles, directement dans le réacteur de fermentation, qu'il s'agisse d'une extraction liquide-liquide ou solide-liquide.

Il est donc possible, dans un mode de réalisation préféré, d'effectuer une fermentation en mode continu avec l'extraction in situ des métabolites inhibiteurs de fermentation, c'est-à-dire en extrayant les acides gras volatils responsables de l'acidose du milieu, au fur et à mesure de leur production ou au moins à intervalles réguliers. En variante non illustrée, ces opérations d'extraction peuvent être réalisées dans un second compartiment. Dans ce cas, un soutirage continu ou à intervalle régulier du milieu de fermentation est réalisé.

La mise en oeuvre d'un tel procédé implique non seulement la présence dans l'installation d'au moins un réacteur de fermentation mais également au moins un organe d'extraction, adapté pour mettre en œuvre l'étape d'extraction. Ces organes sont connus en soi, leurs nombres et leurs dimensions étant adaptées au type de production, selon que l'extraction est faite dans le réacteur ou en dehors.

Une telle installation comprend, avantageusement au moins un organe de stockage des produits issus de l'extraction. Des moyens de gestion et de commande, tels que des capteurs de température, des sondes de pH et/ou de redox, sont prévus. Par ailleurs, le suivi de l'activité des microorganismes est réalisé par des méthodes connues en soi, par exemple par le suivi analytique de la production de métabolites gazeux et liquides, des comptages par cytométrie en flux, des techniques de biologie moléculaire telles que les empreintes moléculaires ou les biopuces.

## Revendications

1. Procédé d'extraction d'acides gras volatils (AGV), molécules organiques dites précurseurs produites par des microorganismes (M) dans un réacteur (2) de fermentation par fermentation (3) anaérobie à partir de biomasse (1) fermentescible, lesdites molécules étant des métabolites fermentaires, **caractérisé en ce qu'**il comprend au moins les étapes suivantes :
a) choisir un moyen d'extraction (8) parmi des moyens d'extraction qui sont, au moins, insolubles dans le milieu de fermentation et dont les conditions de mise en œuvre préservent la capacité des microorganismes (M) présents dans le milieu de fermentation à produire les molécules, le moyen d'extraction (8) est un solvant dont le point d'ébullition est inférieur à 70°C, l'extraction étant de type liquide-liquide et le moyen d'extraction (8) est un solvant ayant un point d'ébullition inférieur à la température de fermentation,
- b) mettre en contact (9) le moyen d'extraction (8) choisi avec le milieu de fermentation, sans interruption de la fermentation (3),
- c) récupérer (12) les molécules extraites, à un pH inférieur à 4,5, par le moyen d'extraction (8) en dehors du réacteur (2) de fermentation,
- d) après l'étape c) au moins une partie de la phase liquide (5) issue de l'extraction (4) est réintroduite (6) dans le réacteur (2) de fermentation et incorporée au milieu de fermentation.

2. Procédé selon la revendication 1, **caractérisé en ce que** le moyen d'extraction (8) est un solvant dont la densité est inférieure à la densité du milieu de fermentation.

3. Procédé selon l'une des revendications précédentes, **caractérisé en ce que** la mise en contact (9) entre le milieu de fermentation et le moyen d'extraction (8) a lieu dans le réacteur (2), le moyen d'extraction étant isolé ou non du milieu de fermentation.

4. Procédé selon la revendication 1 à 2, **caractérisé en ce que** la mise en contact (9) entre le milieu de fermentation et le moyen d'extraction (8) a lieu hors du réacteur (2), le milieu de fermentation étant prélevé en continu.

5. Procédé selon la revendication 1 à 2, **caractérisé en ce que** la mise en contact (9) entre le milieu de fermentation et le moyen d'extraction (8) a lieu hors du réacteur, le milieu de fermentation étant prélevé séquentiellement.

## Patentansprüche

1. Verfahren zur Extraktion flüchtiger Fettsäuren (VFAs, volatile fatty acids), organischer Moleküle, sogenannter Vorläufer, die von Mikroorganismen (M) in einem anaeroben Gärungsreaktor (2) durch anaerobe Gärung (3) aus vergärbarer Biomasse (1) hergestellt werden, wobei die genannten Moleküle Gärungsmetaboliten sind, **dadurch gekennzeichnet, dass** es mindestens die folgenden Schritte umfasst:
a) Wählen eines Extraktionsmittels (8) aus Extraktionsmitteln, die mindestens im Gärungsmedium unlöslich sind und deren Anwendungsbedingungen die Fähigkeit der im Gärungsmedium vorhandenen Mikroorganismen (M) bewahren, die Moleküle herzustellen, wobei das Extraktionsmittel (8) ein Lösungsmittel mit einem Siedepunkt unter 70°C ist, wobei die Extraktion vom Flüssig-Flüssig-Typ ist und das Extraktionsmittel (8) ein Lösungsmittel mit einem Siedepunkt unter der Gärungstemperatur ist,
b) Inkontaktbringen (9) des gewählten Extraktionsmittels (8) mit dem Gärungsmedium, ohne die Gärung (3) zu unterbrechen,
c) Rückgewinnen (12) der extrahierten Moleküle, bei einem pH-Wert unter 4,5, mit dem Extraktionsmittel (8) außerhalb des Gärungsreaktors (2),
d) nach dem Schritt c) erneutes Einleiten (6) mindestens eines Teils der aus der Extraktion (4) hervorgegangenen flüssigen Phase (5) in den Gärungsreaktor (2) und Einbinden in das Gärungsmedium.

2. Verfahren nach Anspruch 1, **dadurch gekennzeichnet, dass** das Extraktionsmittel (8) ein Lösungsmittel ist, dessen Dichte geringer als die Dichte des Gärungsmediums ist.

3. Verfahren nach einem der vorhergehenden Ansprüche, **dadurch gekennzeichnet, dass** das Inkontaktbringen (9) zwischen dem Gärungsmedium und dem Extraktionsmittel (8) im Reaktor (2) stattfindet, wobei das Extraktionsmittel vom Gärungsmedium isoliert wird oder nicht.

4. Verfahren nach Anspruch 1 bis 2, **dadurch gekennzeichnet, dass** das Inkontaktbringen (9) zwischen dem Gärungsmedium und dem Extraktionsmittel (8) außerhalb des Reaktors (2) stattfindet, wobei das Gärungsmedium kontinuierlich entnommen wird.

5. Verfahren nach Anspruch 1 bis 2, **dadurch gekennzeichnet, dass** das Inkontaktbringen (9) zwischen dem Gärungsmedium und dem Extraktionsmittel (8) außerhalb des Reaktors stattfindet, wobei das Gärungsmedium sequenziell entnommen wird.

## Claims

1. A method for extracting volatile fatty acids (VFA), so-called precursor organic molecules produced by microorganisms (M) in a reactor (2) for fermentation by anaerobic fermentation (3) from fermentable biomass (1), said molecules being fermentation metabolites, **characterised in that** the method comprises at least the following steps:
a) choosing an extraction means (8) among extraction means that are, at least, insoluble in the fermentation medium and the implementation conditions of which preserve the ability of the microorganisms (M) in the fermentation medium to produce the molecules, the extraction means (8) being a solvent with a boiling point below 70°C, the extraction being of the liquid-liquid type and the extraction means (8) being a solvent having a boiling point below the fermentation temperature,
b) placing the chosen extraction means (8) in contact (9) with the fermentation medium, without interrupting the fermentation (3),
c) recovering (12) the extracted molecules, at a pH of less than 4.5, by the extraction means (8) outside the fermentation reactor (2),
d) after step c) at least part of the liquid phase (5) from the extraction (4) is reintroduced (6) into the fermentation reactor (2) and incorporated into the fermentation medium.

2. The method according to claim 1, **characterised in that** the extraction means (8) is a solvent, the density of which is less than the density of the fermentation medium.

3. The method according to any one of the preceding claims, **characterised in that** the placing of the extraction means (8) in contact (9) with the fermentation medium takes place in the reactor (2), and the extraction means is isolated or not isolated from the fermentation medium.

4. The method according to claim 1 to 2, **characterised in that** the placing of the extraction means (8) in contact (9) with the fermentation medium takes place outside the reactor (2), and the fermentation medium is withdrawn continuously.

5. The method according to claim 1 to 2, **characterised in that** the placing of the extraction means (8) in contact (9) with the fermentation medium takes place outside the reactor, and the fermentation medium is withdrawn sequentially.
